# EUROPÄISCHE PATENTSCHRIFT

(11) **EP 4 031 611 B1**
(45) Veröffentlichungstag und Bekanntmachung des Hinweises auf die Patenterteilung: **13.11.2024**
(21) Anmeldenummer: 20771322.3
(22) Anmeldetag: 17.09.2020
(51) Int. Cl.: C08J 11/12, C07C 4/22

(54) **VERFAHREN ZUR DEPOLYMERISATION VON POLYSTYROL IN GEGENWART VON FREMDPOLYMEREN**
METHOD FOR THE DEPOLYMERISATION OF POLYSTYRENE IN THE PRESENCE OF FOREIGN POLYMERS
PROCÉDÉ DE DÉPOLYMÉRISATION DE POLYSTYRÈNE EN PRÉSENCE DE POLYMÈRES ÉTRANGERS

(30) Priorität: 18.09.2019 EP 19197997; 10.01.2020 EP 20000008
(43) Veröffentlichungstag der Anmeldung: 27.07.2022
(73) Patentinhaber: INEOS Styrolution Group GmbH, 60325 Frankfurt (DE)
(72) Erfinder: NIESSNER, Norbert, 67159 Friedelsheim (DE); WILHELMUS, Bianca, 63456 Hanau (DE); SCHMIDT-RODENKIRCHEN, Achim, 95445 Bayreuth (DE); MIERDEL, Konstantin, 95444 Bayreuth (DE); NEUNER, Frank, 95448 Bayreuth (DE)
(74) Vertreter: Jacobi, Markus Alexander
(86) Internationale Anmeldenummer: PCT/EP2020/075989
(87) Internationale Veröffentlichungsnummer: WO 2021/053075

(56) Entgegenhaltungen:
- WO-A1-2018/018153
- GUIDO GRAUSE ET AL: "Pyrolysis of Mixed Plastics in a Fluidized Bed of Hard Burnt Lime", INDUSTRIAL & ENGINEERING CHEMISTRY RESEARCH, vol. 50, no. 9, 4 May 2011 (2011-05-04), pages 5459 - 5466, XP055747515, ISSN: 0888-5885, DOI: 10.1021/ie102412h
- W KAMINSKY ET AL: "Pyrolysis of mixed plastics into aromatics", JOURNAL OF ANALYTICAL AND APPLIED PYROLYSIS, vol. 51, no. 1-2, 1 July 1999 (1999-07-01), NL, pages 127 - 134, XP055747508, ISSN: 0165-2370, DOI: 10.1016/S0165-2370(99)00012-1
- KAMINSKY W ET AL: "Feedstock recycling of polymers by pyrolysis in a fluidised bed", POLYMER DEGRADATION AND STABILITY, BARKING, GB, vol. 85, no. 3, 1 September 2004 (2004-09-01), pages 1045 - 1050, XP004524177, ISSN: 0141-3910, DOI: 10.1016/J.POLYMDEGRADSTAB.2003.05.002
- J. AGUADO ET AL: "Fuels from Waste Plastics by Thermal and Catalytic Processes: A Review", INDUSTRIAL & ENGINEERING CHEMISTRY RESEARCH, vol. 47, no. 21, 5 November 2008 (2008-11-05), pages 7982 - 7992, XP055620181, ISSN: 0888-5885, DOI: 10.1021/ie800393w
- SAMIH SAID ET AL: "From complex feedstocks to new processes: The role of the newly developed micro-reactors", CHEMICAL ENGINEERING AND PROCESSING: PROCESS INTENSIFICATION, ELSEVIER SEQUOIA, LAUSANNE, CH, vol. 131, 7 July 2018 (2018-07-07), pages 92 - 105, XP085454186, ISSN: 0255-2701, DOI: 10.1016/J.CEP.2018.07.001

## Beschreibung

Die Erfindung betrifft ein Verfahren zur Herstellung von Styrol-Monomeren durch De-polymerisation (Abbau) von Polystyrol in Gegenwart von Fremdpolymeren, eine Vorrichtung zur Durchführung des Verfahrens sowie die Verwendung einer Vorrichtung zur De-polymerisation von Polystyrol in Gegenwart von Fremdpolymeren.

Polystyrol ist vorzugsweise ausgewählt aus der Gruppe bestehend aus: Standardpolystyrol (GPPS), schlagzähem Polystyrol (HIPS), Styrol-Butadien-Copolymeren. Besonders bevorzugt sind schlagfestes Polystyrol (HIPS) und/oder Standardpolystyrol. Schlagzähe Polystyrole (HIPS) und Standardpolystyrole, sowie deren Herstellung, Struktur und Eigenschaften sind in der Übersichtsliteratur ausführlich beschrieben (A. Echte, et al. in Angew. Chem. (Int. Ed. Engl.) 20, 344-361 (1981); und auch im Kunststoffhandbuch, herausgegeben von R. Vieweg und G. Daumiller, Band 4 "Polystyrol", Carl-Hanser-Verlag München (1996).

Der Übergang von einer Linear- zu einer Kreislaufwirtschaft ist angesichts von Klimawandel, Umweltverschmutzung, Bevölkerungswachstum und Ressourcenabhängigkeit sowohl ökologisch als auch ökonomisch erforderlich. In den 1980er und 1990er Jahren wurden bereits intensive Anstrengungen zur Entwicklung von Verfahren für die rohstoffliche Wiederverwertung von Kunststoffabfällen unternommen, wobei es aufgrund ungelöster verfahrenstechnischer Problemstellungen und aus wirtschaftlichen Gründen bisher nicht zu großtechnischen Anwendungen kam. Aufgrund des allgemein wachsenden Umweltbewusstseins und einem steigenden Bedarf an nachhaltigen Lösungen wächst allerdings auch das Interesse an chemischem Recycling.

Nicht alle thermoplastischen Polymere sind gleichermaßen für das chemische Recycling geeignet. Bei der thermischen Zersetzung von Polyolefinen oder Polyestern entstehen Gemische aus u.a. Wachsen, Leichtöl und Gasen. Der Abbau von Polyethylenterephthalat (PET) führt zu organischen Säuren, hauptsächlich Benzoesäure und Terephthalsäure, die korrosiv sind, und auch eine Verstopfung des Reaktors verursachen können (G. Grause et al., Rohstoffrecycling von polymerem Abfallmaterial, in: Journal of Material Cycles and Waste Management, 13(4), 2011, 265-282). Im Fall von Polystyrol und anderen Styrol-haltigen Polymeren ist es möglich, diese Polymere in ihre Grundbestandteile, insbesondere Styrol-Monomere, zu depolymerisieren, wodurch Polystyrol und andere Styrolmonomer-haltige Polymere eine ausgezeichnete Wahl für das chemische Recycling bilden.

Das resultierende Produktgemisch eines Depolymerisations-Prozesses muss jedoch gereinigt werden, um die Komponenten als Rohmaterial für neue Zwecke, wie Polymerisationsprozesse, zu verwenden.

Wenn Polystyrol ausreichend thermisch behandelt wird, zersetzt es sich zu Styrol-Monomeren, aber eine unvollständige Zersetzung führt auch zur Bildung von z.B. Styrol-Dimeren, -Trimeren und anderen Oligomeren. Wenn die Zersetzungsbedingungen zu harsch sind, können Nebenprodukte, z. Benzol, Toluol, Ethylbenzol, Cumol und alpha-Methylstyrol gebildet werden. Die Mengen dieser Reaktionsprodukte variieren und hängen von den Reaktionsbedingungen und den verwendeten Rohstoffen ab (s. C. Bouster et al., Study of the pyrolysis of polystyrenes: Kinetics of thermal decomposition, Journal of Analytical and Applied Pyrolysis, 1 (1980) 297-313 und C. Bouster et al., Evolution of the product yield with temperature and molecular weight in the pyrolysis of polystyrene, in: Journal of Analytical and Applied Pyrolysis 15 (1989) 249-259).

Die gewonnenen Styrol-Monomere können für ein neues Polymerisationsverfahren verwendet werden. Styrol-Oligomere können ggf. den Polymerisationsprozess stören, da sie bereits in geringen Mengen wichtige Eigenschaften des Polymers beeinflussen. Dies gilt auch für andere Nebenprodukte. Daher müssen die Styrol-Monomere von anderen Komponenten des Produktgemisches getrennt werden, um eine hohe Produktqualität zu gewährleisten.

Insbesondere andere aromatische Verbindungen als Styrol-Monomere können bei radikalischen Polymerisationsverfahren als Kettenübertragungsmittel wirken, die das durchschnittliche Molekulargewicht der hergestellten Polymere senken und zu Polymeren mit einer niedrigeren Glasübergangstemperatur (Tg) beitragen (DS Achilias et al., Chemical recycling of polystyrene by pyrolysis: Potential use of the liquid product for the reproduction of polymer, in: Macromolecular Materials and Engineering, 292(8) (2007), 923-934). Protonen von z.B. Carbonsäuren, Alkoholen, Aldehyden oder Ketonen wirken als Abbruchmittel bei anionischen Polymerisationsverfahren von Styrol (D. Baskaran et al., Anionic Vinyl Polymerization, in: Controlled and Living Polymerizations: From Mechanisms to Applications, John Wiley & Sons, 2009, 1-56).

Im vorliegenden Verfahren kann Polystyrol vorzugsweise ausgewählt aus der Gruppe Standardpolystyrol (GPPS), schlagzähem Polystyrol (HIPS) und Styrol-Butadien-Copolymere eingesetzt werden. Die verwendeten schlagzähen Polystyrole werden durch die Verwendung spezieller Polybutadienkautschuke mit beispielsweise einer modifizierten 1,4-cis- und / oder 1,4-trans-Fraktion oder 1,2- und 1,4-Bindung strukturell modifiziert gegenüber herkömmlichen Kautschuken.

Weiterhin können anstelle von Polybutadienkautschuk auch andere Dien-Kautschuke sowie Elastomere vom Typ Ethylen-Propylen-Dien-Copolymer (EPDM-Kautschuk) sowie hydrierte Dienkautschuke oder auch Siliconkautschuke eingesetzt werden. In dem als Polystyrol verwendeten schlagfesten Polystyrol beträgt der Dienkautschukanteil, insbesondere der Polybutadienkautschuk-Anteil, im allgemeinen 5 bis 12 Gew .-%, bevorzugt 6 bis 10 Gew .-%, besonders bevorzugt 7 bis 9 Gew .-% und der Polystyrolanteil beträgt im allgemeinen 88 bis 95 Gew .-%, bevorzugt 90 bis 94 Gew .-%, bevorzugter 91 bis 93 Gew .-%, wobei die Summe aus Polystyrolanteil und Dienkautschukanteil 100 Gew.-% ergibt.

Geeignetes Standardpolystyrol wird nach der Methode der anionischen oder radikalischen Polymerisation hergestellt. Dabei ist die durch den Polymerisationsprozess beeinflussbare Molekulargewchts-Uneinheitlichkeit des Polymers von untergeordneter Bedeutung. Bevorzugt sind Standardpolystyrol und schlagzähes Polystyrol, dessen toluollöslicher Anteil ein mittleres Molekulargewicht Mw von 150.000 bis 300.000 g / mol, besonders bevorzugt 150.000 bis 270.000 g / mol aufweist, und das gegebenenfalls weiter ausgestattet ist mit Zusatzstoffen, wie beispielsweise Mineralöl (z.B. Weißöl), Stabilisator, Antistatika, Flammschutzmittel oder Wachse.

Erfindungsgemäß als Polystyrol eingesetzte Styrol-Copolymere können auch Methyl(meth)acrylat, α-Methylstyrol und/oder Maleinsäureanhydrid, sowie weitere, copolymerisierbare Monomere enthalten.

In JP 2005132802 und JPH 11100875 (Toshiba Plant Systems) werden Verfahren zur Rückgewinnung von Styrol aus Polystyrol-Abfällen beschrieben, jedoch wird keine Lösung zur Trennung von Leichtsiedern, Styrol und Schwersiedern bereitgestellt.

Es besteht ein hoher Bedarf an einem Verfahren zur Herstellung von Styrol-Monomeren durch De-polymerisation von Polystyrol in Gegenwart von Fremdpolymeren, das Styrol in sehr hoher Ausbeute liefert und dabei die Bildung von Styrol-Oligomeren möglichst gering hält, um den nachfolgenden Reinigungsprozess zu minimieren. Unter Fremdpolymeren werden dabei (Co)Polymere verstanden, die sich von Polystyrol strukturell und in ihren Eigenschaften unterscheiden.

Es ist bekannt, dass das Wirbelschicht-Verfahren zur Depolymerisation von Polystyrol geeignet ist. Liu et al. ("Pyrolysis of polystyrene waste in a fluidized-bed reactor to obtain styrene monomer and gasoline fraction", Fuel Processing Technology, 63, 45-55 (2000)) und Williams et al. ("Product composition from the fast pyrolysis of polystyrene", Environmental Technology, 20, 1109-1118 (1999)) untersuchen die Pyrolyse von PS-Abfällen mittels eines Wirbelschichtreaktors und beobachten dabei die Depolymerisation von Polystyrol in Styrol-Mononere und -Oligomere.

Guido Grause et al.: "Pyrolysis of Mixed Plastics in a Fluidized Bed of Hard Burnt Lime" (Industrial & Engineering Chemistry Research 2011, 50, 5459-5466), W. Kaminsky et al.: "Pyrolysis of mixed plastics into aromatics" (Journal of Analytical and Applied Pyrolysis 1999, 51, 127-134), W. Kaminsky et al: "Feedstock recycling of polymers by pyrolysis in a fluidised bed" (Polymer Degradation and Stability 2004, 85, 1045-1050), Samih Said et al.: "From complex feedstocks to new processes: The role of the newly developed micro-reactors" (Chemical Engineering and Processing: Process Intensification 2018, 131, 92-105) und WO 2018/018153 A1 offenbaren verschiedene Verfahren zur Depolymerisation von Polystyrol-haltigen Kunststoffen in Wirbelschichtreaktoren und anderen Reaktortypen.

In Anwesenheit von Fremdpolymeren besteht die Schwierigkeit, dass diese die De-polymerisation von Polystyrol negativ beeinträchtigen können. Eine Aufgabe der Erfindung ist es daher, ein Verfahren zur Herstellung von Styrol-Monomeren durch De-polymerisation von Polystyrol in Gegenwart von Fremdpolymeren mit optimierten Prozessbedingungen bereitzustellen, um die Ausbeute an Styrol-Monomeren zu maximieren.

Es wurde gefunden, dass bestimmte Kombinationen aus Depolymerisations-Temperatur und Verweilzeit im Reaktor zu besonders hohen Styrol-Monomer-Ausbeuten führen.

Ein Gegenstand der Erfindung ist ein Verfahren zur Herstellung von Styrol-Monomeren durch Depolymerisation einer Polystyrol-haltigen Polymer-Zusammensetzung, umfassend die Schritte:
a) Einbringen einer Polymerzusammensetzung (A) enthaltend:
   I) 60 bis 99,5 Gew.-%, bezogen auf das Gesamtgewicht der Polymerzusammensetzung (A), an Polystyrol (I); und
   II) 0,1 bis 30 Gew.-%, bezogen auf das Gesamtgewicht der Polymerzusammensetzung (A), an Polyolefin (II); und/oder
   III) 0,1 bis 4,9 Gew.-%, bezogen auf das Gesamtgewicht der Polymerzusammensetzung (A), an Acrylnitril-basiertem Polymer (III); und/oder
   IV) 0,1 bis 4,9 Gew.-%, bezogen auf das Gesamtgewicht der Polymerzusammensetzung (A), an Polyester (IV),
   in die Reaktionszone (R) eines Pyrolysereaktors (P);
b) thermisches Spalten des in der Polymerzusammensetzung (A) enthaltenen Polystyrols in der Reaktionszone (R) des Pyrolysereaktors (P) bei einer Temperatur von 450 °C bis 1000 °C, um ein Produktgemisch (G), enthaltend Styrol-Monomere und weitere Komponenten, zu erhalten;
c) Entnehmen des in Schritt b) erhaltenen Produktgemisches (G) aus der Reaktionszone (R) des Pyrolysereaktors (P);
d) Abkühlen des in Schritt c) entnommenen Produktgemisches (G), um ein kondensiertes Produktgemisch (K), enthaltend Styrol-Monomere und weitere Komponenten, zu erhalten; und
e) Abtrennen der Styrol-Monomere von den weiteren Komponenten des in Schritt d) erhaltenen kondensierten Produktgemisches (K),
wobei die mittlere Verweilzeit (Z) der Polymerzusammensetzung (A) in der Reaktionszone (R) des Pyrolysereaktors (P) von 0,01 s bis 10 s beträgt.

Zusätzlich kann die Polymerzusammensetzung (A) bis zu 30 Gew.-%, bezogen auf das Gesamtgewicht der Polymerzusammensetzung (A), an Additiv(en) enthalten.

Als Polyolefin (II) kommen beliebige Polyolefine in Frage, beispielsweise Polyethylen- oder Polypropylen-Derivate wie PE-LD (Low Density Polyethylen), PE-LLD (Linear Low Density Polyethylen), PE-HD (High Density Polyethylen), Metallocen-Polyethylene, Ethylen-Copolymere wie Poly(ethylen-co-vinylacetat), Ethylen-Buten, Ethylen-Hexen, Ethylen-Octen-Copolymere, sowie Cycloolefincopolymere, Homo- oder Copolymere des Propylens, Metallocen-katalysierte Polypropylene sowie Copolymere des Propylens mit anderen, dem Fachmann bekannten Comonomeren, und Mischungen davon. Bevorzugte Polyolefine (II) sind Homopolymeren von Ethylen, Homopolymeren von Propylen, Copolymeren von Ethylen und Propylen und Mischungen davon.

Als Acrylnitril-basiertes Polymer (III) kommen beliebige Acrylnitril-basierte Polymere in Frage, beispielsweise Styrol-Acrylnitril-Copolymere (SAN), α-Methylstyrol-Acrylnitril-Copolymere (AMSAN), Styrol-Acrylnitril-Maleinsäureanhydrid-Copolymere, Styrol-Acrylnitril-Phenylmaleinimid-Copolymere, und deren Pfropfcopolymere mit kautschukartigen Polymeren, z.B. Acrylnitril-Butadien-Styrol-Pfropfcopolymere (ABS), Acrylnitril-Styrol-Alkyl(meth)acrylat-Pfropfcopolymere (ASA), α-Methylstyrol-Acrylnitril-Methylmethacrylat-Copolymere, α-Methylstyrol-Acrylnitril-t-Butylmethacrylat-Copolymere und Styrol-Acrylnitril-t-Butylmethacrylat-Copolymere.

Bevorzugte Acrylnitril-basierte Polymere (III) sind Styrol-Acrylnitril-Copolymere (SAN), Acrylnitril-Styrol-Alkylacrylat-Pfropfcopolymere (ASA) und Acrylnitril-Butadien-Styrol-Pfropfcopolymere (ABS).

Als Polyester (IV) kommen beliebige Polyester in Frage, beispielsweise Polykondensationsprodukte von Dicarbonsäuren enthaltend 4 bis 16 Kohlenstoffatome mit Diolen enthaltend 2 bis 8 Kolenstoffatome oder Polykondensationsprodukte von Hydroxycarbonsäuren enthaltend 2 bis 6 Kohlenstoffatome. Hierzu gehören unter anderem Polyalkylenadipate, wie Polyethylenadipat und Polybutylenadipat, Polyalkylenterephthalate, wie Polyethylenterephthalat und Polybutylenterephthalat, Polymilchsäure, Polyhydroxybutyrate, Polycaprolactone und Polyvalerolactone. Bevorzugte Polyester (IV) sind Polyethylenterephthalat (PET) und Polybutylenterephthalat (PBT), insbesondere Polyethylenterephthalat.

In einer bevorzugten Ausführungsform enthält die Polymerzusammensetzung (A) als Polyolefin (II) Polyethylen (PE) und/oder Polypropylen (PP), und/oder als Acrylnitril-basiertes Polymer (III) Acrylnitril-Butadien-Styrol-Pfropfcopolymer (ABS) und/oder als Polyester (IV) Polyethylenterephthalat (PET).

Erfindungsgemäß enthält die Polymerzusammensetzung (A)
I) 60 bis 99,5 Gew.-%, bezogen auf das Gesamtgewicht der Polymerzusammensetzung (A), an Polystyrol (I); und
II) 0,1 bis 30 Gew.-%, bezogen auf das Gesamtgewicht der Polymerzusammensetzung (A), an Polyolefin (II); und/oder
III) 0,1 bis 4,9 Gew.-%, bezogen auf das Gesamtgewicht der Polymerzusammensetzung (A), an Acrylnitril-basiertem Polymer (III); und/oder
IV) 0,1 bis 4,9 Gew.-%, bezogen auf das Gesamtgewicht der Polymerzusammensetzung (A), an Polyester (IV).

Bevorzugt enthält die Polymerzusammensetzung (A) bis zu 30 Gew.-%, insbesondere 0,1 bis 20 Gew.-%, bezogen auf das Gesamtgewicht der Polymerzusammensetzung (A), an Additiv(en).

In einer bevorzugten Ausführungsform enthält die Polymerzusammensetzung (A) in Summe höchstens 4,9 Gew.-%, bezogen auf das Gesamtgewicht der Polymerzusammensetzung (A), an Acrylnitril-basiertem Polymer (III) und Polyester (IV).

Besonders bevorzugt enthält die Polymerzusammensetzung (A)
I) 85 bis 99,5 Gew.-%, bezogen auf das Gesamtgewicht der Polymerzusammensetzung (A), an Polystyrol (I); und
II) 0,1 bis 15 Gew.-%, bezogen auf das Gesamtgewicht der Polymerzusammensetzung (A), an Polyolefin (II); und/oder
III) 0,1 bis 2 Gew.-%, bezogen auf das Gesamtgewicht der Polymerzusammensetzung (A), an Acrylnitril-basiertem Polymer (III); und/oder
IV) 0,1 bis 2 Gew.-%, bezogen auf das Gesamtgewicht der Polymerzusammensetzung (A), an Polyester (IV).

Besonders bevorzugt enthält die Polymerzusammensetzung (A) bis zu 10 Gew.-%, insbesondere 0,3 bis 8 Gew.-%, bezogen auf das Gesamtgewicht der Polymerzusammensetzung (A), an Additiv(en).

In einer besonders bevorzugten Ausführungsform enthält die Polymerzusammensetzung (A) in Summe höchstens 2 Gew.-%, bezogen auf das Gesamtgewicht der Polymerzusammensetzung (A), an Acrylnitril-basiertem Polymer (III) und Polyester (IV).

Ganz besonders bevorzugt enthält die Polymerzusammensetzung (A)
I) 95 bis 99,5 Gew.-%, bezogen auf das Gesamtgewicht der Polymerzusammensetzung (A), an Polystyrol (I); und
II) 0,1 bis 5 Gew.-%, bezogen auf das Gesamtgewicht der Polymerzusammensetzung (A), an Polyolefin (II); und/oder
III) 0,1 bis 1 Gew.-%, bezogen auf das Gesamtgewicht der Polymerzusammensetzung (A), an Acrylnitril-basiertem Polymer (III); und/oder
IV) 0,1 bis 1 Gew.-%, bezogen auf das Gesamtgewicht der Polymerzusammensetzung (A), an Polyester (IV).

Ganz besonders bevorzugt enthält die Polymerzusammensetzung (A) bis zu 4 Gew.-%, insbesondere 0,3 bis 4 Gew.-%, bezogen auf das Gesamtgewicht der Polymerzusammensetzung (A), Additive.

In einer ganz besonders bevorzugten Ausführungsform enthält die Polymerzusammensetzung (A) in Summe höchstens 1 Gew.-%, bezogen auf das Gesamtgewicht der Polymerzusammensetzung (A), an Acrylnitril-basiertem Polymer (III) und Polyester (IV). Vorzugsweise enthält die Polymerzusammensetzung (A) keine anderen polymeren Komponenten als Polystyrol (I) und Polyolefin (II) und/oder Acrylnitril-basiertes Polymer (III) und/oder Polyester (IV).

Als Additive in der Polymerzusammensetzung (A) können übliche Kunststoffadditive und -Hilfsstoffe enthalten sein. Beispielshaft kann ein Additiv oder ein Hilfsstoff ausgewählt sein aus der Gruppe bestehend aus Antioxidantien, UV-Stabilisatoren, Peroxidzerstörern, Antistatika, Gleitmitteln, Entformungsmitteln, Flammschutzmitteln, Füll- oder Verstärkerstoffen (Glasfasern, Kohlefasern, etc.), Farbmitteln und Kombinationen aus zwei oder mehr daraus.

Als Beispiele für Oxidationsverzögerer und Wärmestabilisatoren seien Halogenide von Metallen der Gruppe I des periodischen Systems, z.B. Natrium-, Kalium- und/oder Lithiumhalogenide, ggf. in Verbindung mit Kupfer-(I)-Halogeniden, z.B. Chloriden, Bromiden, Jodiden, sterisch gehinderte Phenole, Hydrochinone, verschiedene substituierte Vertreter dieser Gruppen und deren Mischungen in Konzentrationen bis zu 1 Gew.-%, bezogen auf das Gesamtgewicht der Polymerzusammensetzung (A) genannt.

Als UV-Stabilisatoren, die im Allgemeinen in Mengen bis zu 2 Gew.-%, bezogen auf das Gesamtgewicht der Polymerzusammensetzung (A) enthalten sind, seien verschiedene substituierte Resorcine, Salicylate, Benzotriazole und Benzophenone genannt.

Weiterhin können organische Farbstoffe wie Nigrosin, Pigmente wie Titandioxid, Phthalocyanine, Ultramarinblau und Ruß als Farbstoffe in der Polymerzusammensetzung (A) enthalten sein, sowie faser- und pulverförmige Füllstoffe und Verstärkungsmittel. Beispiele für letztere sind Kohlenstoffasern, Glasfasern, amorphe Kieselsäure, Calciumsilicat (Wollastonit), Aluminiumsilicat, Magnesiumcarbonat, Kaolin, Kreide, gepulverter Quarz, Glimmer und Feldspat.

Als Keimbildungsmittel können z.B. Talkum, Calciumfluorid, Natriumphenylphosphinat, Aluminiumoxid, Siliciumdioxid und Nylon 22 enthalten sein.

Gleit- und Entformungsmittel, welche in der Regel in Mengen bis zu 1 Gew.-%, bezogen auf das Gesamtgewicht der Polymerzusammensetzung (A), eingesetzt werden können, sind z.B. langkettige Fettsäuren wie Stearinsäure oder Behensäure, deren Salze (z.B. Ca- oder Zn-Stearat) oder Ester (z.B. Stearylstearat oder Pentaerythrittetrastearat) sowie Amidderivate (z.B. Ethylenbisstearylamid).

Weiterhin können Antiblockmittel auf mineralischer Basis in Mengen bis zu 0,1 Gew.-%, bezogen auf das Gesamtgewicht der Polymerzusammensetzung (A), enthalten sein. Als Beispiele seien amorphe oder kristalline Kieselsäure, Calciumcarbonat oder Aluminiumsilikat genannt.

Als Verarbeitungshilfsmittel kann beispielsweise Mineralöl, vorzugsweise medizinisches Weißöl, in Mengen bis zu 5 Gew.-%, vorzugsweise bis zu 2 Gew.-%, insbesondere 0,1 bis 2 Gew.-%, bezogen auf das Gesamtgewicht der Polymerzusammensetzung (A), enthalten sein.

Als Beispiele für Weichmacher seien Phthalsäuredioctylester, Phthalsäuredibenzylester, Phthalsäurebutylbenzylester, Kohlenwasserstofföle, N-(n-Butyl)benzolsulfonamid und o- und p-Tolylethylsulfonamid genannt.

Weiterhin können alle für die jeweiligen Thermoplaste bekannten Flammschutzmittel enthalten sein, insbesondere solche auf Basis von Phosphorverbindungen.

Weiterhin können noch Feuchtigkeit und/oder weitere anorganische und/oder organische Fremdbestandteile, wie z.B. Nahrungsmittelrückstände, enthalten sein.

Bevorzugt besteht die Polystyrol-Komponente (I), die in der Polymerzusammensetzung (A) enthalten ist, im Wesentlichen, vorzugsweise vollständig, aus GPPS (general purpose polystyrene). In diesem Zusammenhang bedeutet "im Wesentlichen", dass in der Polystyrol-Komponente keine Bestandteile enthalten sind, die die Eigenschaften des GPPS in irgendeiner Weise verändern.

Die erfindungsgemäß eingesetzte Polymerzusammensetzung (A) kann gegebenenfalls in geeigneter Weise vorbehandelt werden, um beispielsweise anhaftende Verunreinigungen wie etwa Lebensmittelreste oder Schmutz, Feuchtigkeit und Fremdstoffe wie Metalle oder andere Stoffe und Verbundmaterialien zu entfernen.

Dies erfolgt vorteilhaft in einer Vorbehandlung, die einen oder mehrere der folgenden Schritte umfassen kann, wobei die Reihenfolge der Schritte nicht festgelegt ist und Schritte auch mehrfach wiederholt werden können: manuelle Störstoffsortierung, Wäsche, Zerkleinerung, automatische Sortierung in geeigneten Anlagen. Gegebenenfalls können auch Polymerzusammensetzungen, die der Polymerzusammensetzung (A) nicht entsprechen, durch ein solches Verfahren in eine erfindungsgemäß eingesetzte Polymerzusammensetzung (A) umgewandelt werden.

Bevorzugt ist daher auch eine Ausführungsform des erfindungsgemäßen Verfahrens, bei der eine Polymerzusammensetzung, die der Polymerzusammensetzung (A) nicht entspricht, einer Vorbehandlung unterworfen wird, die einen oder mehrere der folgenden Schritte umfasst, wobei die Reihenfolge der Schritte nicht festgelegt ist und Schritte auch mehrfach wiederholt werden können: manuelle Störstoffsortierung, Wäsche, Zerkleinerung, automatische Sortierung in geeigneten Anlagen, um so die Polymerzusammensetzung (A) zu erhalten.

Die thermische Zersetzung des Polystyrols kann in jedem geeigneten Reaktor stattfinden, in dem die zur Zersetzung erforderliche Temperatur erreicht werden kann. Beispielsweise kann die thermische Zersetzung in einem Drehrohrofen durchgeführt werden. Drehrohröfen sind z.B. in EP-A 1481957 beschrieben. Die thermische Zersetzung kann auch in Extrudern stattfinden; diese sind z.B. in EP-A 1966291 beschrieben. Solche Reaktoren können mit oder ohne Gasstrom betrieben werden, etwa Trägergas oder Gas als Reaktionsmedium.

Der Pyrolysereaktor (P) kann demnach eine beliebige bekannte Art von Pyrolysereaktor sein, unter der Voraussetzung, dass der Aufbau des Pyrolysereaktors eine genaue Einstellung der Temperatur in der Reaktionszone (R) des Pyrolysereaktors (P) und eine genaue Einstellung der mittleren Verweilzeit (Z) der Polymerzusammensetzung (A) in der Reaktionszone (R) des Pyrolysereaktors (P) ermöglicht.

In diesem Zusammenhang bedeutet "genaue Einstellung", dass die Abweichung der tatsächlichen Temperatur in der Reaktionszone (R) des Pyrolysereaktors (P) von der eingestellten Temperatur höchstens 10 °C, bevorzugt höchstens 5 °C, besonders bevorzugt höchstens 1 °C beträgt, bzw. dass die Abweichung der tatsächlichen mittleren Verweilzeit (Z) von der eingestellten mittleren Verweilzeit (Z) höchstens 0,2 s, bevorzugt höchstens 0,1 s, besonders bevorzugt höchstens 0,05 s, ganz besonders bevorzugt höchstens 0,01 s beträgt.

Bei dem Pyrolysereaktor (P) handelt es sich vorzugsweise um einen Wirbelschichtreaktor, beschrieben z.B. in Schildhauer et al., "Reaktoren für Fluid-Feststoff-Reaktionen: Wirbelschichtreaktoren" In: Reschetilowski W. (Hrsg.): Handbuch Chemische Reaktoren, Springer Spektrum, Berlin, Heidelberg (2019). Üblicherweise wird dabei ein Gas oder ein Gasgemisch als Fluidisierungs-Medium verwendet. Das Gas oder Gasgemisch ist üblicherweise nicht-reaktiv bei den im Pyrolysereaktor (P) vorherrschenden Bedingungen. Bevorzugt wird das Gas ausgewählt aus Wasserdampf, Stickstoff, Edelgasen (Xe, Ar, Ne, He) oder einer Mischung daraus.

Das Gas oder Gasgemisch kann als separater Gasstrom in den Pyrolysereaktor (P) eingebracht werden. Die optimale Fließgeschwindigkeit des Gases oder Gasgemisches hängt von der Reaktorkonfiguration sowie von den Reaktions- oder Prozessbedingungen ab. Die Optimierung des Prozesses ist über die Parameter Temperatur, Verweilzeit des Reaktionsgemisches in der Reaktionszone (R) des Pyrolysereaktors (P) und Konzentration der Ausgangsstoffe möglich. Der Bereich des Reaktors, in dem das Reaktionsgemisch solchen Bedingungen unterworfen wird, bei denen Depolymerisationsreaktionen stattfinden, wird hier als Reaktionszone (R) bezeichnet.

Der Pyrolysereaktor (P) kann ein festes Medium enthalten, z.B. SiOz (Quarzsand) oder SiC. In einer bevorzugten Ausführungsform ist der Pyrolysereaktor (P) ein Wirbelschichtreaktor, der in der Reaktionszone (R) eine Wirbelschicht aus Siliciumcarbid (SiC) umfasst. SiC verfügt, neben hoher chemischer und mechanischer Stabilität, über eine gute Wärmeleitung und Wärmekapazität. Diese Kombination ist für die Durchführung endothermer Prozesse vorteilhaft.

Bevorzugt wird SiC mit einer gewichtsmittleren Partikelgröße von 20 bis 1000 µm, besonders bevorzugt 40 bis 500 µm verwendet. Bei solchen Partikelgrößen wird eine besonders gute Fluidisierung des Wirbelbettes erreicht und eine besonders effektive Wärmeübertragung auf die Polymerzusammensetzung (A) ermöglicht.

Es wurde festgestellt, dass die Ausbeute an Styrol-Monomeren besonders hoch ist, wenn die Verweilzeit des Polystyrols in der Depolymerisations-Zone kurz gewählt wird. Dies kann in Wirbelschichtreaktoren oder anderen Reaktoren erreicht werden, die einen Gasstrom zum Transport der eingeführten Polymerzusammensetzung (A) durch den Reaktor nutzen. Die mittleren Verweilzeiten des Reaktionsgutes liegen zwischen 0,1 Sekunden und 10 Sekunden. Kurze Verweilzeiten können durch hohe Fließgeschwindigkeiten des Gasstromes erreicht werden. Die Depolymerisation kann bei Drücken von 0,01 bar bis 5 bar oder bei Atmosphärendruck (1 bar) durchgeführt werden. Bevorzugt wird der Gasstrom vorgeheizt, bevor er in die Reaktionszone (R) geleitet wird. Das Vorheizen erfolgt bevorzugt bei einer Temperatur, die von der Temperatur in der Reaktionszone (R) des Pyrolysereaktors (P) um höchstens 200 °C nach unten abweicht.

Die Temperatur in der Reaktionszone (R) des Pyrolysereaktors (P) wird in dem erfindungsgemäßen Verfahren auf 400 bis 1000 °C, bevorzugt auf 500 bis 800 °C, besonders bevorzugt auf 550 bis 650 °C, ganz besonders bevorzugt auf 580 bis 630 °C, und die mittlere Verweilzeit der Polymerzusammensetzung (A) in der Reaktionszone (R) des Pyrolysereaktors (P) auf 0,1 bis 10 s, bevorzugt auf 0,2 bis 2 s, besonders bevorzugt auf 0,3 bis 1 s, ganz besonders bevorzugt auf 0,4 bis 0,6 s eingestellt. In einer bevorzugten Ausführungsform ist die Temperatur in der Reaktionszone (R) 500 bis 800 °C und die mittlere Verweilzeit der Polymerzusammensetzung (A) darin 0,2 bis 2 s. In einer besonders bevorzugten Ausführungsform ist die Temperatur in der Reaktionszone (R) 550 bis 650 °C und die mittlere Verweilzeit der Polymerzusammensetzung (A) darin 0,3 bis 1 s. In einer ganz besonders bevorzugten Ausführungsform ist die Temperatur in der Reaktionszone (R) 580 bis 630 °C und die mittlere Verweilzeit der Polymer-zusammensetzung (A) darin 0,4 bis 0,6 s.

Die Einstellung der Temperatur kann auf beliebige bekannte Art erfolgen, beispielsweise durch Mikrowelleneinstrahlung, mit Hilfe von Wärmetauschern, Gasbrennern, resistiven Heizleitern (Widerstandsheizung), oder durch Einleiten von überhitztem Gas, insbesondere Wasserdampf, jeweils allein oder in Kombination. In einer Ausführungsform erfolgt die Einstellung der Temperatur mit Hilfe von resistiven Heizleitern, die mit der Wand der Reaktionszone (R) des Pyrolysereaktors (P) in Kontakt stehen.

Alternativ kann die Einstellung der Temperatur mit Hilfe von Wasserdampf erfolgen, der durch Verdampfen von Wasser zur Verfügung gestellt und durch einen Dampfüberhitzer auf die gewünschte Temperatur gebracht wird. In einer Ausführungsform wird eine Kombination von resistiven Heizleitern, die mit der Wand der Reaktionszone (R) des Pyrolysereaktors (P) in Kontakt stehen, und Wasserdampf für die Einstellung der Temperatur in der Reaktionszone (R) des Pyrolysereaktors (P) verwendet.

In einer bevorzugten Ausführungsform ist der Pyrolysereaktor (P) ein Wirbelschichtreaktor, vorzugsweise umfassend eine SiC-Wirbelschicht in dessen Reaktionszone (R), wobei die Temperatur in der Reaktionszone (R) durch Einleiten von Wasserdampf der gewünschten Temperatur eingestellt wird. In dieser Ausführungsform wird der Wasserdampf durch einen Verdampfer zur Verfügung gestellt und über einen Dampfüberhitzer auf die gewünschte Temperatur gebracht. Ebenfalls wird in dieser Ausführungsform der Wasserdampf sowohl zum Einstellen der Temperatur in der Reaktionszone (R) als auch zur Erzeugung der Wirbelschicht verwendet. Optional kann bei dieser Ausführungsform die Heizenergie in der Reaktionszone (R) zusätzlich durch resistive Heizleiter, die mit der Wand der Reaktionszone (R) des Pyrolysereaktors (P) in Kontakt stehen, zur Verfügung gestellt werden.

Die Teilchengröße und die Konzentration der Polymerzusammensetzung (A) in der Reaktionszone (R) können an die spezifischen Gegebenheiten des Reaktors und seiner Konfiguration, an die Reaktionsbedingungen und an die Zusammensetzung der Polymerzusammensetzung (A) angepasst werden. Typischerweise hat die Polymerzusammensetzung (A) eine Teilchengröße zwischen 100 µm und 50 mm, bevorzugt zwischen 250 µm und 5 mm, besonders bevorzugt zwischen 500 µm und 2 mm. Die Teilchen können eine sphärische oder eine nicht-sphärische Form aufweisen. Im Fall einer sphärischen Form wird die Partikelgröße durch Messung des volumenmittleren Durchmessers bestimmt. Im Fall von nicht-sphärischen Partikeln, beispielsweise nadelförmigen Partikeln, wird die längste Dimension zur Bestimmung der Partikelgröße herangezogen.

Die Konzentration des Reaktionsgutes im Reaktor hängt vom Reaktortyp, der Reaktorgröße und der Reaktorkonfiguration ab. Typischerweise liegt die Konzentration der Polymerzusammensetzung (A) in der Reaktionszone (R) des Pyrolysereaktors (P) zwischen 1 und 50 Gew.-%, bevorzugt zwischen 1 und 25 Gew.-%, besonders bevorzugt zwischen 1 und 10 Gew.-%, ganz besonders bevorzugt zwischen 2 und 7 Gew.-%.

Bevorzugt wird die Polymerzusammensetzung (A) in Schritt a) pneumatisch in die Reaktionszone (R) des Pyrolysereaktors (P) zugeführt, mit der Wirbelschicht aus Siliciumcarbid vermischt und schließlich in Schritt b) depolymerisiert. Besonders bevorzugt erfolgt die Zuführung der Polymerzusammensetzung (A) in Schritt a) kontinuierlich.

Während der Verweilzeit (Z) in der Reaktionszone (R) des Pyrolysereaktors (P) wird die Polystyrol-Komponente und gegebenenfalls andere Polymere der Polymerzusammensetzung (A) zumindest teilweise depolymerisiert, um ein Produktgemisch (G) enthaltend Styrol-Monomere und andere Komponenten zu ergeben.

Das Produktgemisch (G) enthaltend Styrol-Monomere und andere Komponenten wird in Schritt c) aus der Reaktionszone (R) des Pyrolysereaktors (P) entnommen. Bevorzugt findet die Entnahme kontinuierlich statt. Besonders bevorzugt wird das Produktgemisch (G) in gasförmigem Zustand aus dem oberen Bereich der Reaktionszone (R) des Pyrolysereaktors (P) kontinuierlich entnommen. Hierbei kann die Entnahme des Produktgemisches (G) beispielsweise durch Beförderung des Produktgemisches (G) aus der Reaktionszone (R) aufgrund des reaktionsbedingt erhöhten Drucks in der Reaktionszone (R) automatisch erfolgen.

Der Pyrolysereaktor kann eine Quensche enthalten oder mit einer Quensche verbunden sein. Unter Quensche wird in diesem Zusammenhang ein Bereich des Pyrolysereaktors verstanden, in dem die Depolymerisationsreaktion schnell abgestoppt wird, bevorzugt durch Abkühlen des heißen Gases bestehend aus dem Produktgemisch (G) und inertem Gasstrom, beispielsweise Wasserdampf. Die Quensche dient unter anderem zur Stabilisierung der Reaktionsprodukte und zur Verhinderung oder Verminderung einer unerwünschten Repolymerisation.

Typische Quenschen kühlen das Produktgemisch (G) von einer Temperatur von mehr als 400 °C auf eine Temperatur von unter 350 °C innerhalb eines sehr kurzen Zeitraums ab, bevorzugt innerhalb von weniger als 10 Sekunden, besonders bevorzugt innerhalb von weniger als 5 Sekunden, besonders bevorzugt innerhalb von weniger als 1 Sekunde. Quenschen sind z.B. beschrieben in EP-A 1966291.

Das Produktgemisch (G) wird nach der Entnahme aus der Reaktionszone (R) des Pyrolysereaktos (P) abgekühlt, wodurch eine Kondensation der Styrol-Monomere und weiterer Komponenten stattfindet und ein kondensiertes Produktgemisch (K) enthaltend Styrol-Monomere und weitere Komponenten erhalten wird. Das Produktgemisch (G) wird auf eine Temperatur abgekühlt, die unter dem Kondensationspunkt von Styrol-Monomeren liegt. Bevorzugt wird das Produktgemisch (G) auf eine Temperatur unter 70 °C, besonders bevorzugt unter 50 °C, ganz besonders bevorzugt unter 40 °C gekühlt. In einer bevorzugten Ausführungsform wird das Produktgemisch (G) auf eine Temperatur von 0 °C bis 70 °C gekühlt. In einer besonders bevorzugten Ausführungsform wird das Produktgemisch (G) auf eine Temperatur von 0 °C bis 50 °C gekühlt. In einer ganz besonders bevorzugten Ausführungsform wird das Produktgemisch (G) auf eine Temperatur von 15 °C bis 40 °C gekühlt.

Die Abkühlung kann auf eine beliebige bekannte Art und Weise erfolgen. Beispielsweise ist die Abkühlung an einer festen Oberfläche möglich, die durch Wasser oder Luft gekühlt wird. Ebenfalls ist die Abkühlung durch einen Wassernebel möglich, der direkt mit dem Produktgemisch (G) in Kontakt gebracht wird. Bevorzugt findet die Abkühlung in einem Wassernebel statt. Dabei werden die kondensierbaren Bestandteile des Produktgemischs (G) entsprechend ihren Dampfdrücken kondensiert und zusammen mit dem Wasser aufgefangen. Bei der Kondensation wird ein kondensiertes Produktgemisch (K) enthaltend Styrol-Monomere und weitere Bestandteile erhalten.

Im Falle der Abkühlung in einem Wassernebel wird das kondensierte Produktgemisch (K) als zwei-Phasen-System mit dem Kühlwasser erhalten. In diesem Fall wird das kondensierte Produktgemisch (K) als aufschwimmende organische Phase von der wässrigen Phase getrennt. Die wässrige Phase kann erneut gekühlt und als Wassernebel zum Abkühlen des Produktgemischs (G) eingesetzt werden.

Das kondensierte Produktgemisch (K) enthält üblicherweise mehr als 50 Gew.-%, bezogen auf das Gesamtgewicht des kondensierten Produktgemischs (K), Styrol-Monomere und weniger als 50 Gew.-%, bezogen auf das Gesamtgewicht des kondensierten Produktgemischs (K), der weiteren Bestandteile.

Vorzugsweise enthält das kondensierte Produktgemisch (K) 50 bis 99 Gew.-%, bezogen auf das Gesamtgewicht des kondensierten Produktgemischs (K), Styrol-Monomere und 1 bis 50 Gew.-%, bezogen auf das Gesamtgewicht des kondensierten Produktgemischs (K), der weiteren Bestandteile, besonders vorzugsweise 60 bis 99 Gew.-%, bezogen auf das Gesamtgewicht des kondensierten Produktgemischs (K), Styrol-Monomere und 1 bis 40 Gew.-%, bezogen auf das Gesamtgewicht des kondensierten Produktgemischs (K), der weiteren Bestandteile, ganz besonders vorzugsweise 75 bis 99 Gew.-%, bezogen auf das Gesamtgewicht des kondensierten Produktgemischs (K), Styrol-Monomere und 1 bis 25 Gew.-%, bezogen auf das Gesamtgewicht des kondensierten Produktgemischs (K), der weiteren Bestandteile.

Im weiteren Verlauf des Verfahrens wird das kondensierte Produktgemisch (K) in Styrol-Monomere und weitere Bestandteile getrennt. Diese Trennung kann auf beliebige bekannte Weise durchgeführt werden, die geeignet ist, Mischungen aus flüssigen Produkten und gegebenenfalls Feststoffen in die Bestandteile aufzutrennen. Abhängig von den weiteren Bestandteilen neben Styrol-Monomeren können geeignete Methoden zum Trennen des kondensierten Produktgemischs (K) in Styrol-Monomere und weitere Bestandteile beispielsweise Sedimentation, Zentrifugation, Filtration, Dekantieren, Destillation, Chromatographie, Kristallisation und Sublimation umfassen.

Sind in der kondensierten Produktmischung (K) Feststoffe enthalten, ist es vorteilhaft, vor der Trennung der flüssigen Bestandteile die Feststoffe abzutrennen. In diesem Fall ist es bevorzugt, den Feststoff durch Sedimentation, Zentrifugation, Filtration, Destillation, Sublimation und/oder Dekantieren, besonders bevorzugt durch Filtration, ganz besonders bevorzugt durch Filtration mittels Fest-Flüssig-Filter von den flüssigen Bestandteilen abzutrennen.

Die weitere Auftrennung der flüssigen Bestandteile in Styrol-Monomere und weitere Bestandteile umfasst vorzugsweise mindestens einen Schritt der Destillation, wie zum Beispiel fraktionierende Destillation, mindestens einen Schritt der Chromatographie, wie zum Beispiel Säulenchromatographie, HPLC oder Flash-Chromatographie, und/oder mindestens einen Schritt der Kristallisation, wie zum Beispiel fraktionierende Kristallisation. Besonders vorzugsweise umfasst die Auftrennung der flüssigen Bestandteile mindestens einen Schritt der Destillation, ganz besonders vorzugsweise mindestens einen Schritt der fraktionierenden Destillation. In einer ganz besonders bevorzugten Ausführungsform umfasst die Auftrennung der flüssigen Bestandteile in Styrol-Monomere und weitere Komponenten mindestens einen Schritt der fraktionierenden Destillation in einer oder mehreren Rektifikationskolonnen.

Häufig ist es vorteilhaft, zumindest einen Teil der weiteren Komponenten in die Reaktionszone (R) des Pyrolysereaktors (P) zurückzuführen. Dies ist insbesondere vorteilhaft, wenn ein Teil der weiteren Bestandteile Styrol-Oligomere wie z.B. Styrol-Dimere und Styrol-Trimere enthält. Dies ermöglicht eine insgesamt bessere Ausbeute an Styrol-Monomeren, da hierdurch auch die Depolymerisation der übrig gebliebenen Oligomere ermöglicht wird.

Aus diesem Grund wird vorzugsweise zumindest ein Teil der weiteren Komponenten des kondensierten Produktgemischs (K), die von Styrol-Monomeren abgetrennt wurden, in die Reaktionszone (R) des Pyrolysereaktors (P) rückzuführen. Besonders bevorzugt ist hierbei eine kontinuierliche Rückführung der weiteren Komponenten.

In einer bevorzugten Ausführungsform bestehen die weiteren Komponenten, die in die Reaktionszone (R) des Pyrolysereaktors (P) zurückgeführt werden, im Wesentlichen aus Styrol-Oligomeren, vorzugsweise aus Styrol-Dimeren und Styrol-Trimeren. In diesem Zusammenhang bedeutet "im Wesentlichen", dass die weiteren Komponenten, die in die Reaktionszone (R) des Pyrolysereaktors (P) zurückgeführt werden, neben Styrol-Oligomeren keine weiteren Komponenten enthalten, die den Depolymerisationsprozess in der Reaktionszone (R) des Pyrolysereaktors (P) stören.

Ein weiterer Gegenstand der Erfindung ist die Verwendung eines Wirbelschichtreaktors zur Depolymerisation einer Polystyrol-haltigen Polymerzusammensetzung (A), bei einer Temperatur in der Reaktionszone (R) des Reaktors von 400 °C bis 1000 °C und einer mittleren Verweilzeit der Polystyrol-haltigen Polymerzusammensetzung (A) in der Reaktionszone (R) von 0,01 s bis 10 s, wobei die Polymerzusammensetzung (A) bei Eintritt in die Reaktionszone (R) des Reaktors enthält:
I) 60 bis 99,5 Gew.-%, bezogen auf das Gesamtgewicht der Polymerzusammensetzung (A), an Polystyrol (I); und
II) 0,1 bis 30 Gew.-%, bezogen auf das Gesamtgewicht der Polymerzusammensetzung (A), an Polyolefin (II); und/oder
III) 0,1 bis 4,9 Gew.-%, bezogen auf das Gesamtgewicht der Polymerzusammensetzung (A), an Acrylnitril-basiertem Polymer (III); und/oder
IV) 0,1 bis 4,9 Gew.-%, bezogen auf das Gesamtgewicht der Polymerzusammensetzung (A), an Polyester (IV).

Vorzugsweise umfasst der Wirbelschichtreaktor in der Reaktionszone (R) eine Wirbelschicht aus Siliciumcarbid (SiC). Besonders bevorzugt sind die Polymerzusammensetzung (A), das Siliciumcarbid, die Temperatur in der Reaktionszone (R) und die mittlere Verweilzeit des Polystyrols in der Reaktionszone (R) so gewählt, wie für das erfindungsgemäße Verfahren beschrieben ist.

Zum Durchführen des erfindungsgemäßen Verfahrens geeignet ist eine Vorrichtung zur Durchführung des erfindungsgemäßen Verfahrens zur Herstellung von Styrol-Monomeren durch Depolymerisation einer Polystyrol-haltigen Polymerzusammensetzung (A) wie oben beschrieben, bei der die Reaktionszone (R) und der Pyrolysereaktor (P) so gestaltet sind, dass eine schonende Depolymerisation von Polystyrol erfolgen kann.

Vorzugsweise umfasst die Vorrichtung als Pyrolysereaktor (P) einen Wirbelschichtreaktor, besonders vorzugsweise einen Wirbelschichtreaktor umfassend eine Wirbelschicht aus Siliciumcarbid (SiC) in der Reaktionszone (R), wobei das Siliciumcarbid bevorzugt eine gewichtsmittleren Partikelgröße von 20 bis 1000 µm, besonders bevorzugt 40 bis 500 µm aufweist, optional resistive Heizleiter an den Außenwänden der Reaktionszone (R), einen Verdampfer zum Erzeugen von Wasserdampf, einen Dampfüberhitzer zum Einstellen der gewünschten Dampftemperatur, eine Leitung zum Einführen des Dampfs in die Reaktionszone (R) und eine Quensche zum Abkühlen des Produktgemischs (G).

Ein weiterer Gegenstand der Erfindung ist die Verwendung einer Polymerzusammensetzung (A) enthaltend
I) 60 bis 99,5 Gew.-%, bezogen auf das Gesamtgewicht der Polymerzusammensetzung (A), an Polystyrol (I); und
II) 0,1 bis 30 Gew.-%, bezogen auf das Gesamtgewicht der Polymerzusammensetzung (A), an Polyolefin (II); und/oder
III) 0,1 bis 4,9 Gew.-%, bezogen auf das Gesamtgewicht der Polymerzusammensetzung (A), an Acrylnitril-basiertem Polymer (III); und/oder
IV) 0,1 bis 4,9 Gew.-%, bezogen auf das Gesamtgewicht der Polymerzusammensetzung (A), an Polyester (IV),
als Ausgangsmaterial bei der Herstellung von Styrol-Monomeren durch Depolymerisation in einem Wirbelschichtreaktor.

Bevorzugt sind die Polymerzusammensetzung (A), das Siliciumcarbid, die Temperatur in der Reaktionszone (R) und die mittlere Verweilzeit des Polystyrols in der Reaktionszone (R) so gewählt, wie für das erfindungsgemäße Verfahren beschrieben ist.

Die Erfindung wird durch die folgenden Beispiele, Figuren und Ansprüche illustriert.

### Beispiele

Aus einem inertisierten Vorlagebehälter (B101) wird das Polymer über eine volumetrische Dosierung in einen Gasjet dosiert, welcher die Partikel pneumatisch in die Reaktionszone eines Wirbelschichtreaktors (R101) mit SiC-Partikeln fördert. Die Heizenergie wird über resistive Heizleiter durch die metallische Reaktorwand in die Reaktionszone gebracht.

Der Dampf für die Erzeugung der Wirbelschicht wird durch einen Verdampfer (D101) zur Verfügung gestellt und über einen Dampfüberhitzer (E102) auf die gewünschte Temperatur gebracht.

In der Reaktionszone wird mit dem Dampf die SiC-Wirbelschicht erzeugt und die pneumatisch eingeförderten Polymerpartikel in dem Wirbelbett vermischt und schließlich depolymerisiert.

Das Produktgemisch des Depolymerisations-Prozesses aus (R101) wird in der Kolonne (K101) in einem Wassernebel auf unter 50 °C gekühlt. Dabei werden die kondensierbaren Bestandteile entsprechend ihren Dampfdrücken kondensiert und mit dem Wasser im gekühlten Sumpf aufgefangen. Feststoffpartikel werden ebenfalls entsprechend ihrer Koaleszenz-Neigung mit dem Nebel niedergeschlagen. Aufschwimmende, nicht wasserlösliche Bestandteile werden über ein Skimmersystem in einen Pufferbehälter überführt. Von der aufkonzentrierten, organischen Fraktion des kondensierten Produktgemischs werden Proben abgefüllt. Diese werden mittels Gaschromatographie vermessen.

Das Wasser aus dem Kondensationsprozess wird über einen Wärmetauscher gekühlt und anschließend mit der Pumpe wieder in die Kühlkolonne (K101) geführt. Feststoffe werden gegebenenfalls über einen Fest-Flüssig-Filter abgetrennt.

### Beispiele 1.1 und 1.2

Eine Polymerzusammensetzung bestehend aus 99 Gew.-% eines amorphen Polystyrols (GPPS) (INEOS Styrolution, Frankfurt) mit einer Schmelze-Volumenfließrate (Melt Volume Rate 200 /5 kg Belastung, ISO 1133) von etwa 28 cm³/10 min und 1 Gew.-% Acrylnitril-Butadien-Styrol-Pfropfpolymer (ABS der INEOS Styrolution, Frankfurt) mit einer Schmelze-Volumenfließrate (Melt Volume Rate 220 /10 kg Belastung, ISO 1133) von etwa 5,5 cm³/10 min wurde in der oben beschriebenen Vorrichtung unter verschiedenen Bedingungen depolymerisiert.

Hierbei wurde die Temperatur in der Reaktionszone des Wirbelschichtreaktors (R101) in dem Bereich 550 °C bis 650 °C variiert. Die Konzentration der Polymerzusammensetzung in der Reaktionszone wurde bei 5% bezogen auf die Gesamtfüllung des Reaktors, bestehend aus SiC- und Polymerpartikeln,gehalten. Die Ausbeuten an Styrol-Monomeren abhängig von den Reaktionsbedingungen sind in der Tabelle 1 dargestellt. Die Zusammensetzungen des Pyrolyseöls sind in Tabelle 2 dargestellt.

### Vergleichsbeispiel 1

Beispiel 1 wurde mit einer Polymerzusammensetzung bestehend aus 95 Gew.-% eines amorphen Polystyrols (GPPS) (INEOS Styrolution, Frankfurt) mit einer Schmelze-Volumenfließrate (Melt Volume Rate 200 /5 kg Belastung, ISO 1133) von etwa 28 cm³/10 min und 5 Gew.-% Acrylnitril-Butadien-Styrol-Pfropfpolymer (INEOS Styrolution, Frankfurt) mit einer Schmelze-Volumenfließrate (Melt Volume Rate 220 /10 kg Belastung, ISO 1133) von etwa 5,5 cm³/10 min wiederholt. Die Ausbeute an Styrol-Monomeren ist in der Tabelle 1 dargestellt. Die Zusammensetzung des Pyrolyse-Öls (Kondensat-Öls) ist in Tabelle 2 dargestellt.

### Vergleichsbeispiel 2

Beispiel 1 wurde mit einer Polymerzusammensetzung bestehend aus 100 Gew.-% eines amorphen Polystyrols (GPPS) (INEOS Styrolution, Frankfurt) mit einer Schmelze-Volumenfließrate (Melt Volume Rate 200 /5 kg Belastung, ISO 1133) von etwa 28 cm³/10 min wiederholt. Die Ausbeute an Styrol-Monomeren ist in der Tabelle 1 dargestellt. Die Zusammensetzung des Pyrolyseöls ist in Tabelle 2 dargestellt.

### Beispiel 2

Beispiel 1 wurde mit einer Polymerzusammensetzung bestehend aus 95 Gew.-% eines amorphen Polystyrols (GPPS) (INEOS Styrolution, Frankfurt) mit einer Schmelze-Volumenfließrate (Melt Volume Rate 200 /5 kg Belastung, ISO 1133) von etwa 28 cm³/10 min und 5 Gew.-% Polyethylen (LDPE, Typ 2102, Sabic) wiederholt. Die Ausbeute an Styrol-Monomeren ist in der Tabelle 1 dargestellt. Die Zusammensetzung des Pyrolyseöls ist in Tabelle 2 dargestellt.

### Beispiel 3

Beispiel 1 wurde mit einer Polymerzusammensetzung bestehend aus 90 Gew.-% eines amorphen Polystyrols (GPPS) (INEOS Styrolution, Frankfurt) mit einer Schmelze-Volumenfließrate (Melt Volume Rate 200 /5 kg Belastung, ISO 1133) von etwa 28 cm³/10 min und 10 Gew.-% Polyethylen (LDPE, Typ 2102, Sabic) wiederholt.

Die Ausbeute an Styrol-Monomeren ist in der Tabelle 1 dargestellt. Die Zusammensetzung des Pyrolyseöls ist in Tabelle 2 dargestellt.

### Beispiel 4

Beispiel 1 wurde mit einer Polymerzusammensetzung bestehend aus 95 Gew.-% eines amorphen Polystyrols (GPPS) (INEOS Styrolution, Frankfurt) mit einer Schmelze-Volumenfließrate (Melt Volume Rate 200 /5 kg Belastung, ISO 1133) von etwa 28 cm³/10 min und 5 Gew.-% Polypropylen (Typ 579s, Sabic) wiederholt. Die Ausbeute an Styrol-Monomeren ist in der Tabelle 1 dargestellt. Die Zusammensetzung des Pyrolyseöls ist in Tabelle 2 dargestellt.

### Beispiel 5

Beispiel 1 wurde mit einer Polymerzusammensetzung bestehend aus 90 Gew.-% eines amorphen Polystyrols (GPPS) (INEOS Styrolution, Frankfurt) mit einer Schmelze-Volumenfließrate (Melt Volume Rate 200 /5 kg Belastung, ISO 1133) von etwa 28 cm³/10 min und 10 Gew.-% Polypropylen (Typ 579s, Sabic) wiederholt. Die Ausbeute an Styrol-Monomeren ist in der Tabelle 1 dargestellt. Die Zusammensetzung des Pyrolyseöls ist in Tabelle 2 dargestellt.

### Beispiel 6

Beispiel 1 wurde mit einer Polymerzusammensetzung bestehend aus 95 Gew.-% eines amorphen Polystyrols (GPPS) (INEOS Styrolution, Frankfurt) mit einer Schmelze-Volumenfließrate (Melt Volume Rate 200 /5 kg Belastung, ISO 1133) von etwa 28 cm³/10 min und 5 Gew.-% Titandioxid wiederholt. Die Ausbeute an Styrol-Monomeren ist in der Tabelle 1 dargestellt. Die Zusammensetzung des Pyrolyseöls ist in Tabelle 2 dargestellt.

### Beispiel 7

Beispiel 1 wurde mit einer Polymerzusammensetzung bestehend aus 98 Gew.-% eines amorphen Polystyrols (GPPS) (INEOS Styrolution, Frankfurt) mit einer Schmelze-Volumenfließrate (Melt Volume Rate 200 /5 kg Belastung, ISO 1133) von etwa 28 cm³/10 min und 2 Gew.-% Ruß wiederholt.

Die Ausbeute an Styrol-Monomeren ist in der Tabelle 1 dargestellt. Die Zusammensetzung des Pyrolyseöls ist in Tabelle 2 dargestellt.

**Tabelle 1: Ergebnisse der Pyrolyse der Polymerzusammensetzungen**

| Beispiel | Polymer | Temperatur in der Reaktionszone [°C] | Mittlere Verweilzeit in der Reaktionszone [s] | Masseanteil des Kondensatöls bezogen auf die eingesetzte Masse [Gew.-%] | Styrolkonzentration im Kondensatöl [Gew.-%] |
|---|---|---|---|---|---|
| 1.1 | 99% GPPS / 1% ABS | 550 | 0,51 | 55,6 | 69,8 |
| 1.2 | 99% GPPS / 1% ABS | 600 | 0,51 | 56,7 | 71,6 |
| 2 | 95% GPPS / 5% LDPE | 600 | 0,51 | 56,4 | 73,9 |
| 3 | 90% GPPS / 10% LDPE | 600 | 0,51 | 66,8 | 72,8 |
| 4 | 95% GPPS / 5% PP | 600 | 0,51 | 85,9 | 71,9 |
| 5 | 90% GPPS / 10% PP | 600 | 0,51 | 85,1 | 73,6 |
| 6 | 95% GPPS / 5% TiO₂ | 600 | 0,51 | 41,6 | 77,5 |
| 7 | 98% GPPS / 2% Ruß | 600 | 0,51 | 76,5 | 72,8 |
| Vergleichsbeispiel 1 | 95% GPPS / 5% ABS | 600 | 0,51 | 0,0 | entfällt |
| Vergleichsbeispiel 2 | 100% GPPS | 600 | 0,51 | 65,9 | 83,2 |

**Tabelle 2: Zusammensetzung des erhaltenen Pyrolyse-Öls**

| Beispiel | Styrol-konzentration im Kondensat-öl [Gew.-%] | Dimerkonzentration im Kondensat-öl [Gew.-%] | Trimerkonzentration im Kondensat-öl [Gew.-%] | Konzentration sonstiger Verbindungen im Kondensatöl [Gew.-%] |
|---|---|---|---|---|
| 1.1 | 69,8 | 7,3 | 9,7 | 13,2 |
| 1.2 | 71,6 | 9,2 | 0,6 | 18,6 |
| 2 | 73,9 | 8,7 | 0,7 | 16,8 |
| 3 | 72,8 | 8,1 | 0,6 | 18,5 |
| 4 | 71,9 | 8,1 | 0,6 | 19,4 |
| 5 | 73,6 | 7,3 | 0,5 | 18,7 |
| 6 | 77,5 | 8,0 | 0,4 | 14,1 |
| 7 | 72,8 | 8,7 | 0,6 | 17,8 |
| Vergleich 1 | Kein Pyrolyse-Öl entstanden | Kein Pyrolyse-Öl entstanden | Kein Pyrolyse-Öl entstanden | Kein Pyrolyse-Öl entstanden |
| Vergleich 2 | 83,2 | 6,0 | 0,6 | 10,3 |

### Erklärung der Figuren

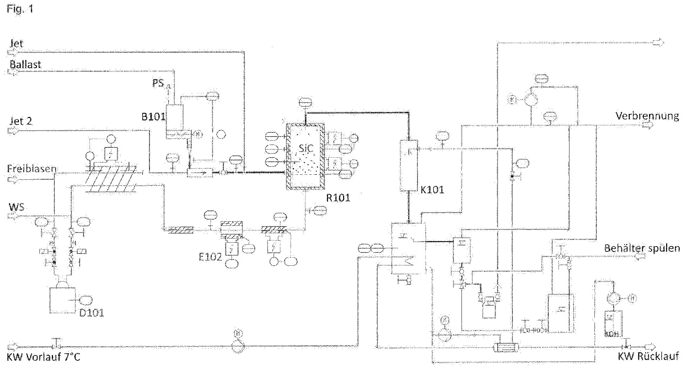

Fig. 1 zeigt Fließbild der Technikums-Anlage zur Herstellung von Styrol-Monomeren durch Depolymerisation von Polystyrol-haltigen Polymer-Zusammensetzungen

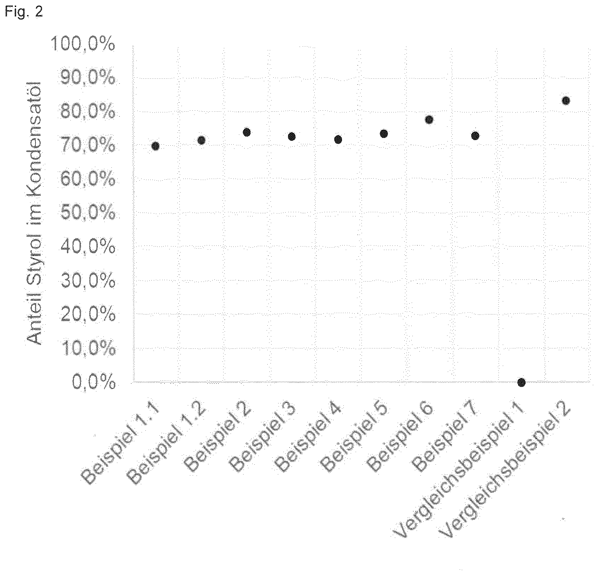

Fig. 2 zeigt eine Übersicht über die Styrol-Konzentration im Kondensat-Öl.

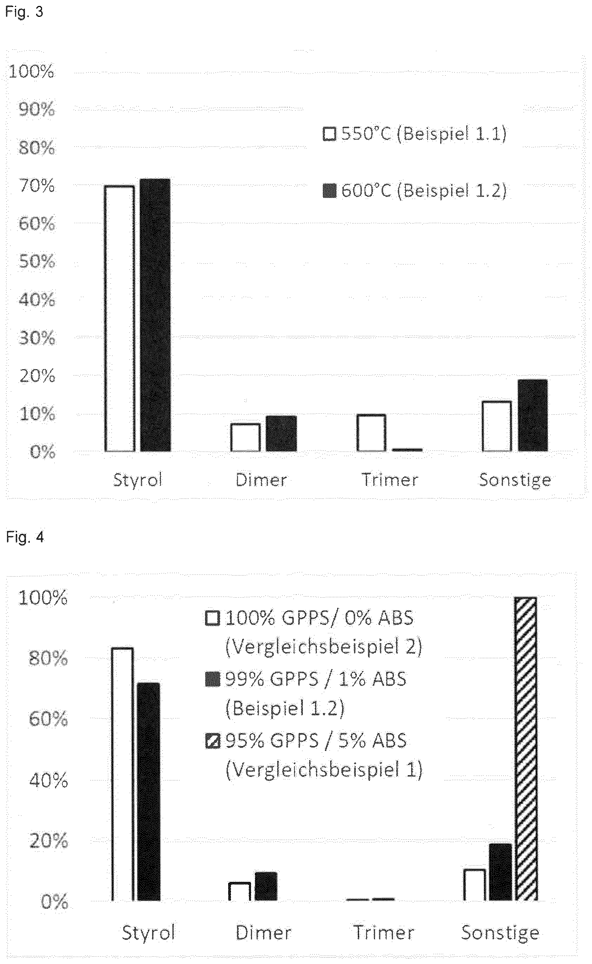

Fig. 3 zeigt die Abhängigkeit der Pyrolyse-Öl-Zusammensetzung von der Temperatur für eine Polymerzusammensetzung von 99 Gew.-% GPPS und 1 Gew.-% ABS.
Fig. 4 zeigt die Abhängigkeit der Pyrolyseöl-Zusammensetzung von der Polymerzusammensetzung für einen ABS-Gehalt von 0, 1 und 5 Gew.-%.

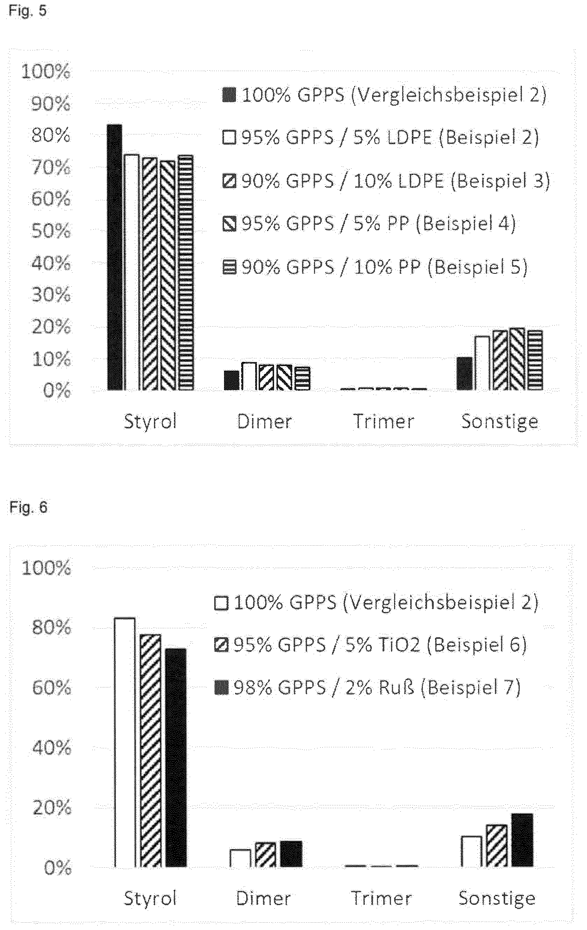

Fig. 5 zeigt die Abhängigkeit der Pyrolyseöl-Zusammensetzung von der Polymerzusammensetzung bei 0, 5 und 10 Gew.-% LDPE und/oder PP.
Fig. 6 zeigt die Abhängigkeit der Pyrolyseöl-Zusammensetzung von der Polymerzusammensetzung bei Zusatz von TiOz bzw. Ruß.

## Patentansprüche

1. Verfahren zur Herstellung von Styrol-Monomeren durch Depolymerisation einer Polystyrol-haltigen Polymer-Zusammensetzung, umfassend die Schritte:
a) Einbringen einer Polymerzusammensetzung (A) enthaltend:
I) 60 bis 99,5 Gew.-%, bezogen auf das Gesamtgewicht der Polymerzusammensetzung (A), an Polystyrol (I); und
II) 0,1 bis 30 Gew.-%, bezogen auf das Gesamtgewicht der Polymerzusammensetzung (A), an Polyolefin (II); und/oder
III) 0,1 bis 4,9 Gew.-%, bezogen auf das Gesamtgewicht der Polymerzusammensetzung (A), an Acrylnitril-basiertem Polymer (III); und/oder
IV) 0,1 bis 4,9 Gew.-%, bezogen auf das Gesamtgewicht der Polymerzusammensetzung (A), an Polyester (IV),
in die Reaktionszone (R) eines Pyrolysereaktors (P);
b) thermisches Spalten des in der Polymerzusammensetzung (A) enthaltenen Polystyrols in der Reaktionszone (R) des Pyrolysereaktors (P) bei einer Temperatur von 400 °C bis 1000 °C, um ein Produktgemisch (G), enthaltend Styrol-Monomere und weitere Komponenten, zu erhalten;
c) Entnehmen des in Schritt b) erhaltenen Produktgemisches (G) aus der Reaktionszone (R) des Pyrolysereaktors (P);
d) Abkühlen des in Schritt c) entnommenen Produktgemisches (G), um ein kondensiertes Produktgemisch (K), enthaltend Styrol-Monomere und weitere Komponenten, zu erhalten; und
e) Abtrennen der Styrol-Monomere von den weiteren Komponenten des in Schritt
d) erhaltenen kondensierten Produktgemisches (K),
wobei die mittlere Verweilzeit (Z) der Polymerzusammensetzung (A) in der Reaktionszone (R) des Pyrolysereaktors (P) von 0,01 s bis 10 s beträgt.

2. Verfahren gemäß Anspruch 1, wobei das Polyolefin (II) Polyethylen (PE) und/oder Polypropylen (PP) ist, und/oder das Acrylnitril-basierte Polymer (III) Acrylnitril-Butadien-Styrol-Pfropfcopolymer (ABS) ist und/oder der Polyester (IV) Polyethylenterephthalat (PET) ist.

3. Verfahren gemäß Anspruch 1 oder 2, wobei die Polymerzusammensetzung (A) das Polystyrol (I) in einer Menge von 85 bis 99,5 Gew.-%, bezogen auf das Gesamtgewicht der Polymerzusammensetzung (A), und das Polyolefin (II) in einer Menge von 0,1 bis 15 Gew.-%, bezogen auf das Gesamtgewicht der Polymerzusammensetzung (A), und/oder das Acrylnitril-basierte Polymer (III) in einer Menge von 0,1 bis 2 Gew.-%, bezogen auf das Gesamtgewicht der Polymerzusammensetzung (A), und/oder den Polyester IV) in einer Menge von 0,1 bis 2 Gew.-%, bezogen auf das Gesamtgewicht der Polymerzusammensetzung (A), enthält.

4. Verfahren gemäß einem der Ansprüche 1 bis 3, wobei die Temperatur in der Reaktionszone (R) des Pyrolysereaktors (P) 580 °C bis 630 °C beträgt und die mittlere Verweilzeit (Z) der Polymerzusammensetzung (A) in der Reaktionszone des Pyrolysereaktors 0,4 s bis 0,6 s beträgt.

5. Verfahren gemäß einem der Ansprüche 1 bis 4, wobei das in Schritt d) erhaltene kondensierte Produktgemisch (K) von 75 bis 99 Gew.-% Styrol und 1 bis 25 Gew.-% der weiteren Komponenten enthält, jeweils bezogen auf das Gesamtgewicht des Produktgemisches (K).

6. Verfahren gemäß einem der Ansprüche 1 bis 5, wobei der Pyrolysereaktor (P) ein Wirbelschichtreaktor, bevorzugt umfassend eine Siliciumcarbid-Wirbelschicht in der Reaktionszone (R) ist.

7. Verfahren gemäß einem der Ansprüche 1 bis 6, wobei zumindest ein Teil der in Schritt e) erhaltenen weiteren Komponenten in die Reaktionszone (R) des Pyrolysereaktors (P) rückgeführt wird.

8. Verfahren gemäß Anspruch 7, bei dem die im Schritt e) erhaltenen weiteren Komponenten, die in die Reaktionszone (R) des Pyrolysereaktors (P), bevorzugt kontinuierlich, rückgeführt werden, im Wesentlichen aus Styrol-Oligomeren bestehen.

9. Verfahren gemäß einem der Ansprüche 1 bis 8, bei dem die Polystyrol-Komponente in der Polymerzusammensetzung (A), die in Schritt a) in die Reaktionszone (R) des Pyrolysereaktors (R) rückgeführt wird, im Wesentlichen aus GPPS (general purpose polystyrene) besteht.

10. Verfahren gemäß einem der Ansprüche 1 bis 9, bei dem in Schritt d) das in Schritt c) entnommene Produktgemisch (G) auf eine Temperatur von 0 °C bis 50 °C gekühlt wird.

11. Verfahren gemäß einem der Ansprüche 1 bis 10, bei dem das Abtrennen der Styrol-Monomere in Schritt e) den Schritt der fraktionierenden Destillation umfasst.

12. Verwendung eines Wirbelschichtreaktors zur Depolymerisation einer Polystyrol-haltigen Polymerzusammensetzung (A), bei einer Temperatur in der Reaktionszone (R) des Reaktors von 400 °C bis 1000 °C und einer mittleren Verweilzeit der Polystyrol-haltigen Polymer-zusammensetzung (A) in der Reaktionszone (R) von 0,01 s bis 10 s, wobei die Polymerzusammensetzung (A) bei Eintritt in die Reaktionszone (R) des Reaktors enthält:
I) 60 bis 99,5 Gew.-%, bezogen auf das Gesamtgewicht der Polymerzusammensetzung (A), an Polystyrol (I); und
II) 0,1 bis 30 Gew.-%, bezogen auf das Gesamtgewicht der Polymerzusammensetzung (A), an Polyolefin (II); und/oder
III) 0,1 bis 4,9 Gew.-%, bezogen auf das Gesamtgewicht der Polymerzusammensetzung (A), an Acrylnitril-basiertem Polymer (III); und/oder
IV) 0,1 bis 4,9 Gew.-%, bezogen auf das Gesamtgewicht der Polymerzusammensetzung (A), an Polyester (IV).

13. Verwendung gemäß Anspruch 12, bei der der Wirbelschichtreaktor eine Siliciumcarbid-Wirbelschicht umfasst.

14. Verwendung einer Polymerzusammensetzung (A) enthaltend
I) 60 bis 99,5 Gew.-%, bezogen auf das Gesamtgewicht der Polymerzusammensetzung (A), an Polystyrol (I); und
II) 0,1 bis 30 Gew.-%, bezogen auf das Gesamtgewicht der Polymerzusammensetzung (A), an Polyolefin (II); und/oder
III) 0,1 bis 4,9 Gew.-%, bezogen auf das Gesamtgewicht der Polymerzusammensetzung (A), an Acrylnitril-basiertem Polymer (III); und/oder
IV) 0,1 bis 4,9 Gew.-%, bezogen auf das Gesamtgewicht der Polymerzusammensetzung (A), an Polyester (IV),
als Ausgangsmaterial bei der Herstellung von Styrol-Monomeren durch Depolymerisation in einem Wirbelschichtreaktor.

## Claims

1. A process for the production of styrene monomers by depolymerization of a polystyrene-containing polymer composition, comprising the steps of:
a) introducing a polymer composition (A) containing:
I) 60% to 99.5% by weight of polystyrene (I), based on the total weight of the polymer composition (A); and
II) 0.1% to 30% by weight of polyolefin (II), based on the total weight of the polymer composition (A); and/or
III) 0.1% to 4.9% by weight of acrylonitrile-based polymer (III), based on the total weight of the polymer composition (A); and/or
IV) 0.1% to 4.9% by weight of polyester (IV), based on the total weight of the polymer composition (A),
into the reaction zone (R) of a pyrolysis reactor (P);
b) thermally cleaving the polystyrene present in the polymer composition (A) in the reaction zone (R) of the pyrolysis reactor (P) at a temperature of from 400°C to 1000°C, in order to obtain a product mixture (G) containing styrene monomers and further components;
c) withdrawing the product mixture (G) obtained in step b) from the reaction zone (R) of the pyrolysis reactor (P);
d) cooling the product mixture (G) withdrawn in step c), in order to obtain a condensed product mixture (K) containing styrene monomers and further components; and
e) separating the styrene monomers from the further components of the condensed product mixture (K) obtained in step d),
wherein the average residence time (Z) of the polymer composition (A) in the reaction zone (R) of the pyrolysis reactor (P) is from 0.01 s to 10 s.

2. The process according to claim 1, wherein the polyolefin (II) is polyethylene (PE) and/or polypropylene (PP), and/or the acrylonitrile-based polymer (III) is acrylonitrile-butadiene-styrene graft copolymer (ABS) and/or the polyester (IV) is polyethylene terephthalate (PET).

3. The process according to claim 1 or 2, wherein the polymer composition (A) contains the polystyrene (I) in an amount of from 85% to 99.5% by weight, based on the total weight of the polymer composition (A), and the polyolefin (II) in an amount of from 0.1% to 15% by weight, based on the total weight of the polymer composition (A), and/or the acrylonitrile-based polymer (III) in an amount of from 0.1% to 2% by weight, based on the total weight of the polymer composition (A), and/or the polyester (IV) in an amount of from 0.1% to 2% by weight, based on the total weight of the polymer composition (A).

4. The process according to any one of claims 1 to 3, wherein the temperature in the reaction zone (R) of the pyrolysis reactor (P) is 580°C to 630°C and the average residence time (Z) of the polymer composition (A) in the reaction zone of the pyrolysis reactor is 0.4 s to 0.6 s.

5. The process according to any one of claims 1 to 4, wherein the condensed product mixture (K) obtained in step d) contains from 75% to 99% by weight of styrene and 1% to 25% by weight of the further components, based in each case on the total weight of the product mixture (K).

6. The process according to any one of claims 1 to 5, wherein the pyrolysis reactor (P) is a fluidized bed reactor, preferably comprising a silicon carbide fluidized bed in the reaction zone (R).

7. The process according to any one of claims 1 to 6, wherein at least a portion of the further components obtained in step e) is recycled into the reaction zone (R) of the pyrolysis reactor (P).

8. The process according to claim 7, in which the further components obtained in step e) which are recycled, preferably continuously, into the reaction zone (R) of the pyrolysis reactor (P) essentially consist of styrene oligomers.

9. The process according to any one of claims 1 to 8, in which the polystyrene component in the polymer composition (A), which in step a) is recycled into the reaction zone (R) of the pyrolysis reactor (R), essentially consists of GPPS (general purpose polystyrene).

10. The process according to any one of claims 1 to 9, in which, in step d), the product mixture (G) withdrawn in step c) is cooled to a temperature of from 0°C to 50°C.

11. The process according to any one of claims 1 to 10, in which the separation of the styrene monomers in step e) comprises a step of fractional distillation.

12. The use of a fluidized bed reactor for the depolymerization of a polystyrene-containing polymer composition (A), at a temperature in the reaction zone (R) of the reactor of from 400°C to 1000°C and with an average residence time of the polystyrene-containing polymer composition (A) in the reaction zone (R) of from 0.01 s to 10 s, wherein the polymer composition (A) on entry into the reaction zone (R) of the reactor contains:
I) 60% to 99.5% by weight of polystyrene (I), based on the total weight of the polymer composition (A); and
II) 0.1% to 30% by weight of polyolefin (II), based on the total weight of the polymer composition (A); and/or
III) 0.1% to 4.9% by weight of acrylonitrile-based polymer (III), based on the total weight of the polymer composition (A); and/or
IV) 0.1% to 4.9% by weight of polyester (IV), based on the total weight of the polymer composition (A).

13. The use according to claim 12, wherein the fluidized bed reactor comprises a silicon carbide fluidized bed.

14. The use of a polymer composition (A) containing
I) 60% to 99.5% by weight of polystyrene (I), based on the total weight of the polymer composition (A); and
II) 0.1% to 30% by weight of polyolefin (II), based on the total weight of the polymer composition (A); and/or
III) 0.1% to 4.9% by weight of acrylonitrile-based polymer (III), based on the total weight of the polymer composition (A); and/or
IV) 0.1% to 4.9% by weight of polyester (IV), based on the total weight of the polymer composition (A),
as a feedstock for the production of styrene monomers by depolymerization in a fluidized bed reactor.

## Revendications

1. Procédé de préparation de monomères du styrène par dépolymérisation d'une composition de polymères contenant un polystyrène, comprenant les étapes :
a) introduction, dans la zone de réaction (R) d'un réacteur de pyrolyse (P), d'une composition de polymères (A) contenant :
I) 60 à 99,5 % en poids, par rapport au poids total de la composition de polymères (A), d'un polystyrène (I) ; et
II) 0,1 à 30 % en poids, par rapport au poids total de la composition de polymères (A), d'une polyoléfine (II) ; et/ou
III) 0,1 à 4,9 % en poids, par rapport au poids total de la composition de polymères (A), d'un polymère à base d'acrylonitrile (III) ; et/ou
IV) 0,1 à 4,9 % en poids, par rapport au poids total de la composition de polymères (A), d'un polyester (IV) ;
b) dissociation thermique du polystyrène contenu dans la composition de polymères (A) dans la zone de réaction (R) du réacteur de pyrolyse (P) à une température de 400 °C à 1 000 °C pour obtenir un mélange de produits (G), contenant des monomères du styrène et d'autres composants ;
c) prélèvement, de la zone de réaction (R) du réacteur de pyrolyse (P), du mélange de produits (G) obtenu dans l'étape b) ;
d) refroidissement du mélange de produits (G) prélevé dans l'étape c) pour obtenir un mélange de produits condensé (K) contenant des monomères du styrène et d'autres composants ; et
e) séparation des monomères du styrène des autres composants du mélange de produits condensé (K) obtenu dans l'étape d),
le temps de séjour moyen (Z) de la composition de polymères (A) dans la zone de réaction (R) du réacteur de pyrolyse (P) étant de 0,01 s à 10 s.

2. Procédé selon la revendication 1, dans lequel la polyoléfine (II) est un polyéthylène (PE) et/ou un polypropylène (PP), et/ou le polymère à base d'acrylonitrile (III) est un copolymère greffé acrylonitrile-butadiène-styrène (ABS) et/ou le polyester (IV) est un poly(téréphtalate d'éthylène) (PET) .

3. Procédé selon la revendication 1 ou 2, dans lequel la composition de polymères (A) contient le polystyrène (I) en une quantité de 85 à 99,5 % en poids par rapport au poids total de la composition de polymères (A), et la polyoléfine (II) en une quantité de 0,1 à 15 % en poids, par rapport au poids total de la composition de polymères (A) et/ou le polymère à base d'acrylonitrile (III) en une quantité de 0,1 à 2 % en poids par rapport au poids total de la composition de polymères (A), et/ou le polyester IV) en une quantité de 0,1 à 2 % en poids par rapport au poids total de la composition de polymères (A).

4. Procédé selon l'une des revendications 1 à 3, dans lequel la température dans la zone de réaction (R) du réacteur de pyrolyse (P) est de 580 °C à 630 °C et le temps de séjour moyen (Z) de la composition de polymères (A) dans la zone de réaction du réacteur de pyrolyse est de 0,4 s à 0,6 s.

5. Procédé selon l'une des revendications 1 à 4, dans lequel le mélange de produits condensé (K) obtenu dans l'étape d) contient de 75 à 99 % en poids de styrène et 1 à 25 % en poids des autres composants, dans chaque cas par rapport au poids total du mélange de produits (K) .

6. Procédé selon l'une des revendications 1 à 5, dans lequel le réacteur de pyrolyse (P) est un réacteur à lit fluidisé, comprenant de préférence un lit fluidisé de carbure de silicium dans la zone de réaction (R).

7. Procédé selon l'une des revendications 1 à 6, dans lequel au moins une partie des autres composants obtenus dans l'étape e) sont renvoyés dans la zone de réaction (R) du réacteur de pyrolyse (P).

8. Procédé selon la revendication 7, dans lequel les autres composants obtenus dans l'étape e), qui sont renvoyés, de préférence en continu, dans la zone de réaction (R) du réacteur de pyrolyse (P), sont pour l'essentiel constitués d'oligomères du styrène.

9. Procédé selon l'une des revendications 1 à 8, dans lequel le composant polystyrène de la composition de polymères (A), qui est renvoyé dans l'étape a) dans la zone de réaction (R) du réacteur de pyrolyse (R), est pour l'essentiel constitué de GPPS (polystyrène à usage général) .

10. Procédé selon l'une des revendications 1 à 9, dans lequel dans l'étape d) le mélange de produits (G) prélevé dans l'étape c) est refroidi à une température de 0 °C à 50 °C.

11. Procédé selon l'une des revendications 1 à 10, dans lequel la séparation des monomères du styrène dans l'étape e) comprend l'étape de distillation fractionnée.

12. Utilisation d'un réacteur à lit fluidisé pour la dépolymérisation d'une composition de polymères (A) contenant un polystyrène, à une température dans la zone de réaction (R) du réacteur de 400 °C à 1 000 °C et pour un temps de séjour moyen de la composition de polymères (A) contenant du polystyrène dans la zone de réaction (R) de 0,01 s à 10 s, la composition de polymères (A) contenant, lors de son entrée dans la zone de réaction (R) du réacteur :
I) 60 à 99,5 % en poids, par rapport au poids total de la composition de polymères (A), d'un polystyrène (I) ; et
II) 0,1 à 30 % en poids, par rapport au poids total de la composition de polymères (A), d'une polyoléfine (II) ; et/ou
III) 0,1 à 4,9 % en poids, par rapport au poids total de la composition de polymères (A), d'un polymère à base d'acrylonitrile (III) ; et/ou
IV) 0,1 à 4,9 % en poids, par rapport au poids total de la composition de polymères (A), d'un polyester (IV) .

13. Utilisation selon la revendication 12, dans laquelle le réacteur à lit fluidisé comprend un lit fluidisé de carbure de silicium.

14. Utilisation d'une composition de polymères (A) contenant
I) 60 à 99,5 % en poids, par rapport au poids total de la composition de polymères (A), d'un polystyrène (I) ; et
II) 0,1 à 30 % en poids, par rapport au poids total de la composition de polymères (A), d'une polyoléfine (II) ; et/ou
III) 0,1 à 4,9 % en poids, par rapport au poids total de la composition de polymères (A), d'un polymère à base d'acrylonitrile (III) ; et/ou
IV) 0,1 à 4,9 % en poids, par rapport au poids total de la composition de polymères (A), d'un polyester (IV),
comme matière de départ lors de la fabrication de monomères du styrène par dépolymérisation dans un réacteur à lit fluidisé.
